# EUROPÄISCHE PATENTSCHRIFT

(11) **EP 2 746 756 B1**
(45) Veröffentlichungstag und Bekanntmachung des Hinweises auf die Patenterteilung: **11.10.2017**
(21) Anmeldenummer: 14160557.6
(22) Anmeldetag: 16.01.2013
(51) Int. Cl.: G01N 27/02, G01N 27/22, A47L 15/42, D06F 39/00, G01N 33/18, G01N 27/30

(54) **Sensorvorrichtung zur Erfassung von Flüssigkeitseigenschaften**
Sensor device for detecting fluid properties
Dispositif de capteur destiné à déterminer les propriétés d'un liquide

(30) Priorität: 20.01.2012 DE 202012000569 U
(43) Veröffentlichungstag der Anmeldung: 25.06.2014
(62) Teilanmeldung aus: 13151477.0
(73) Patentinhaber: Seuffer GmbH & Co. KG, 75365 Calw (DE)
(72) Erfinder: Gruden, Roman, 75180 Pforzheim (DE)
(74) Vertreter: Winter, Brandl, Fürniss, Hübner, Röss, Kaiser, Polte - Partnerschaft mbB

(56) Entgegenhaltungen:
- WO-A2-00/63682
- WO-A2-2011/064770
- DE-A1- 4 236 421
- DE-A1- 10 212 494
- DE-A1-102011 017 547
- DE-T5-112005 000 168
- US-A1- 2005 247 114
- US-A1- 2006 105 467
- US-B1- 7 143 637

## Beschreibung

Die vorliegende Erfindung umfasst eine Sensorvorrichtung zur Erfassung von Flüssigkeitseigenschaften, und insbesondere eine Vorrichtung zur Erfassung von Eigenschaften wässriger Lösungen.

Aus einem Stand der Technik gemäß der Druckschrift DE 196 11 174 C1 ist ein Messwertaufnehmer zur elektrodenlosen Leitfähigkeitsmessung von wässrigen Lösungen bekannt, bei dem drei Transformatoren verwendet werden. Ein erster Transformator steht in Verbindung mit einer Messflüssigkeit und transformiert eine Generatorwechselspannung in ein Magnetfeld, wobei die Messflüssigkeit von dem durch den ersten Transformator mit der Generatorwechselspannung erzeugten Magnetfeld durchdrungen wird. Es sind ferner ein zweiter und ein dritter Transformator vorgesehen, die beide über das in der Flüssigkeit vorhandene Magnetfeld mit dem ersten Transformator verkoppelt sind. Jeder der beiden weiteren Transformatoren mit der Funktion als Ausgangstransformator wird auf eine unterschiedliche Resonanzfrequenz abgeglichen, sodass die beiden Resonanzfrequenzen gegeneinander versetzt sind. Die Addition der beiden Ausgangsspannungen ergibt einen breitbandigen Frequenzgang unter dem Material-einfluss der Messflüssigkeit. Die Ausgangssignale der beiden weiteren Transformatoren werden ausgewertet zur Bestimmung der Leitfähigkeit der Messflüssigkeit in Form einer wässrigen Lösung, wobei mittels einer entsprechenden Schaltungsanordnung eingekoppelte Störsignale beseitigt werden.

Die Druckschrift DD 217 557 A1 offenbart ein Verfahren zur Regelung der Reinigungs- oder Spülmittelzugabe in Waschgeräten, wobei in einem Waschgerät zur physikalischchemischen Bestimmung der Eigenschaften der Waschlauge unter-schiedliche Sensoren angeordnet sind. Über eine elektronische Schaltung, mittels der die Ausgangssignale der Sensoren ausgewertet werden, wird eine Änderung des Anstiegs als Messsignal während der Zugabe von Reinigungs- oder Spülmitteln erfasst und ausgewertet. Es kann hieraus die Waschmitteldosierung für das Waschgerät gesteuert werden.

Schließlich offenbart die Druckschrift DE 197 55 418 A1 ein Sensorelement sowie eine Vorrichtung zur Messung komplexer Impedanzen in Materialien, wobei das Sensorelement zwei aus einem leitfähigen Material bestehende Elektroden aufweist, die in einem vorbestimmten Abstand zueinander angeordnet sind. Die beiden Elektroden sind mit einer dünnen Isolierschicht überzogen, die eine im Vergleich zu dem vorbestimmten Abstand geringe Schichtdicke aufweist. Das gebildete Sensorelement ist gegenüber den Umgebungsmedien, deren Eigenschaften erfasst werden sollen, weitgehend unempfindlich. In einer Auswerteschaltung werden die Ausgangssignale der Sensoren einer weiteren Verarbeitung unterzogen, und es kann eine Analyse der Eigenschaften einer jeweils zwischen den Elektroden angeordneten Flüssigkeit durchgeführt werden. Insbesondere können komplexe Impedanzen als Maß für die Eigenschaften der Flüssigkeit bestimmt und ausgewertet werden.

Entsprechend den vorstehend angegebenen bekannten Möglichkeiten zur Erfassung der Eigenschaften von beispielsweise einer Waschlauge oder sonstigen fluiden Medien können mit den dargestellten Vorrichtungen und einer vergleichs-weise komplizierten Auswertung von Messergebnissen Parameter der Eigenschaften der zu untersuchenden Medien bestimmt werden. Insbesondere besteht allgemein ein großer Bedarf an der Erfassung von Materialeigenschaften wie beispielsweise einer Ölqualität in einer Brennkraftmaschine, und gezielt einen Ölwechsel bei verschlechterten Eigenschaften des verwendeten Öls vorzunehmen. Es kann ebenfalls eine Bremsflüssigkeit in Kraftfahrzeugen oder ein allgemeines Hydrauliköl hinsichtlich seiner im betrieb veränderlichen Eigenschaften überprüft werden.

Die Druckschrift US 2006/0105467 A1 offenbart eine mikroelektronische Sensoreinrichtung zur Erfassung von Eigenschaften von Schmierstoffen, wobei mehrere einzelne Sensoranordnungen auf einem gemeinsamen Substrat vorgesehen sind. Die Sensoreinrichtungen auf der Basis von Interdigital-Anordnungen dienen dazu, Ablagerungen der zu überprüfenden Stoffe zu ermitteln und zu analysieren. Die Sensoreinrichtungen können auf dem gemeinsamen Substrat in gleichartiger Weise ausgeführt sein oder können auch bei gleicher Grundgröße in unterschiedlicher Ausführung vorgesehen sein. Die unterschiedlich ausgeführten Sensoreinrichtungen sind geeignet, unterschiedlich Ablagerungen, d. h. Ablagerungen mit unterschiedlicher Partikelgröße zur Analyse aufzunehmen. Hierzu haben die Elektroden der jeweiligen Sensoreinrichtungen unterschiedliche Abstände, und es ergeben sich in Verbindung mit den verschiedenen Ablagerungen unterschiedliche Impedanzen. Partikel verschiedener Größe werden durch benachbart zu den Sensoreinrichtungen ausgebildete Magnetfelder angezogen.
Die Druckschrift US 7 143 637 B1 offenbart eine dielektrische Chip-Anordnung zur Erfassung von Eigenschaften einer strömenden Flüssigkeit mit unterschiedlichen technischen Maßnahmen, wobei auf der Chip-Anordnung eine Interdigitalstruktur eines kapazitiven Sensors ausgebildet ist. Die gesamte Sensoranordnung kann hierbei eine Vielzahl unterschiedlicher Sensoreinrichtungen zur Erfassung einer Vielzahl von Parametern der strömenden Flüssigkeit aufweisen. Die einzelnen Elektroden der interdigitalen Sensoranordnung sind auf einem gemeinsamen Substrat ausgebildet, wobei ein elektrisches Feld durch das Anlegen einer jeweiligen Spannung gebildet werden kann. Durch ein entsprechendes Erfassen einer zugehörigen Stromantwort in Verbindung mit einer jeweiligen Phasenlage eines Stroms relativ zur Spannung kann auf die Eigenschaften der Flüssigkeit geschlossen werden. Auf demselben Substrat können unterschiedlich ausgebildete Interdigitalstrukturen vorgesehen sein. Die DE 42 36 421 A1 offenbart ferner eine Anordnung aus einer Vielzahl von Sensorelementen, welche zur Messung der Konzentrationen unterschiedlicher Stoffe unterschiedliche geometrische Abmessungen bei gleicher Grundgröße aufweisen.

Schließlich zeigt die DE 10 2011 017547 A1 eine Sensoranordnung zur Messung von Partikeln in Gasen, wobei die Größe von zwei Sensorelementen in vorbestimmter Weise abgestuft ist, um unterschiedliche Erfassungsbereiche zu ermöglichen.

Die vorstehend angegebenen bekannten Möglichkeiten zur Erfassung der Eigenschaften von Flüssigkeiten, wie beispielsweise einer Waschlauge oder sonstigen wässrigen Lösungen, stellen Vorrichtungen und Systeme dar, mittels denen in Verbindung mit einer vergleichsweise komplizierten Auswertung der Messergebnisse und aufwändigen Sensoreinrichtungen die Eigenschaften oder zumindest einzelne Parameter der jeweiligen zu untersuchenden Flüssigkeiten bestimmt werden.

Im Hinblick auf das Bestreben zu einem umweltfreundlichen und gleichzeitig optimierten Waschvorgang besteht allgemein ein großer Bedarf an der Erfassung der Eigenschaften einer Waschlauge in einer Wascheinrichtung zu Beginn und im gesamten Verlauf des Waschvorgangs, sodass in Richtung einer geringeren Umweltverschmutzung beispielsweise die eingesetzte Wassermenge und/oder Waschmittelmenge genau bestimmt und ferner die Waschmittelmenge in Abhängigkeit von dem Verschmutzungsgrad zum Reinigen der Wäsche oder bestimmten Wassereigenschaften (Mineralgehalt, Wasserhärte) genau dosiert werden kann.

Eine gezielte und richtige Waschmitteldosierung führt einerseits zur Einsparung von Energie und Wasser, sowie andererseits zu der erstrebten geringeren Belastung von Abwässern. Eine Erfassungsvorrichtung zur Erfassung einer Waschlauge in einer Wascheinrichtung, beispielsweise hinsichtlich der Waschmittelkonzentration oder eines Verschmutzungsgrads, sollte einfach aufgebaut und betriebssicher ausgeführt sein. Da zukünftig immer mehr Wascheinrichtungen in privaten Haushalten und in der Industrie mit einer Erfassungsvorrichtung zur Erfassung der Eigenschaften der Waschlauge ausgerüstet werden sollen, besteht ein großer Bedarf an einfach aufgebauten Sensorvorrichtungen, die in größeren Stückzahlen kostengünstig hergestellt werden können, und die gleichwohl ein verlässliches Erfassungsergebnis zur weiteren Auswertung bereitstellen können. Im Idealfall sollte jede Wascheinrichtung mit einer derartigen Erfassungseinrichtung oder Sensorvorrichtung ausgerüstet werden, sodass auch die Erfordernisse einer unkomplizierten Montage während des Herstellungsvorgangs der Wascheinrichtung in Verbindung mit dem einfachen Aufbau zu berücksichtigen sind.

Der vorliegenden Erfindung liegt daher die Aufgabe zugrunde, eine Sensorvorrichtung zur Erfassung von Flüssigkeitseigenschaften derart auszugestalten, dass bei einem einfachen Aufbau und einer damit verbundenen kostengünstigen Herstellung eine verlässliche Erfassung der Eigenschaften einer Flüssigkeit gewährleistet ist.

Erfindungsgemäß wird diese Aufgabe mit den in den Patentansprüchen angegebenen Merkmalen gelöst.

Die Erfindung betrifft somit eine Sensorvorrichtung zur Erfassung von Eigenschaften fluider Medien in einem Behälter mit zumindest einer aus einem isolierenden Material gebildeten Grundplatte mit einer ersten den Medien ausgesetzten Oberfläche, einer Mehrzahl gleichartiger Sensorelemente mit jeweils zumindest einer ersten und einer zweiten Elektrode, die gegeneinander isoliert auf der ersten Oberfläche der Grundplatte angeordnet sind und von den Medien umströmt werden, einer Ansteuerungseinrichtung zur Ansteuerung der Sensorelemente, wobei die mehreren Sensorelemente in einer vorbestimmten räumlichen Lage zueinander und auf der ersten Oberfläche verteilt angeordnet sind und jedes Sensorelement über individuelle Anschlussleitungen ansteuerbar ist, die mehreren Sensorelemente in ihren geometrischen Abmessungen unterschiedlich ausgeführt sind, und wobei die Größe der unterschiedlich ausgeführten Sensorelemente in vorbestimmter Weise abgestuft ist, und die Ansteuerungseinrichtung ausgebildet ist, sämtliche Sensorelemente oder Gruppen der Sensorelemente zur Erfassung der Eigenschaften der fluiden Medien anzusteuern.

Die erfindungsgemäße Sensorvorrichtung zur Erfassung von Materialeigenschaften, und im vorliegenden Fall zur Erfassung von Eigenschaften einer Flüssigkeit, wie beispielsweise einer Waschlauge oder anderen wässrigen Lösungen, ist im Wesentlichen als ein kapazitiver Sensor gemäß den Angaben in den Patentansprüchen aufgebaut.

Entsprechende Elektroden sind auf einer Seite einer isolierenden Grundplatte zueinander beabstandet und isoliert angeordnet.

Mit der erfindungsgemäßen Anordnung der Sensorvorrichtung ist es möglich, unterschiedliche Eigenschaften eines interessierenden Mediums, wie beispielsweise einer Waschlauge und anderer wässriger Lösungen in zuverlässiger Weise zu erfassen, wobei die Sensorvorrichtung gleichzeitig einen einfachen Aufbau aufweist. Mittels des einfachen Aufbaus besteht die Möglichkeit einer kostengünstigen Herstellung. Dies ist insbesondere bei der Fertigung der Sensorvorrichtung in größeren Stückzahlen für eine Serie von Wascheinrichtungen oder weiteren Anwendungen vorteilhaft.

Die Sensorvorrichtung ermöglicht die genaue Erfassung von Eigenschaften von wässrigen Lösungen, und kann in Folge der kostengünstigen Herstellung auch bei größeren Stückzahlen beispielsweise in Wascheinrichtungen für private Haushalte und die Industrie eingesetzt werden. Es ist ebenfalls möglich, mittels der erfindungsgemäßen Sensorvorrichtung eine Veränderung der Eigenschaften des interessierenden fluiden Mediums, wie beispielsweise Frischwasser (Mineralgehalt, Wasserhärte) oder auch von Abwässern (sich ändernder Verschmutzungsgrad) zu erfassen. Verschiedene Ausführungsformen werden verschiedenen Anwendungen oder Einsatzbereichen gerecht. Es besteht mit diesen Sensorvorrichtungen die Möglichkeit, gleichzeitig oder in vorbestimmter Weise sequenziell mehrere Eigenschaften (interessierende Parameter) des fluiden Mediums zu erfassen. Allen Ausführungsformen liegt jedoch ein Aufbau zugrunde, der bei einer verlässlichen Erfassung kostengünstig hergestellt und eingesetzt werden kann.

Es können die einzelnen Sensorelemente aus einem gleichen oder unterschiedlichen leitenden Material gebildet sein.

Die Erfindung wird nachstehend anhand von Ausführungsbeispielen unter Bezugnahme auf die Zeichnung näher beschrieben. Es zeigen:
Figur 1 ein Blockschaltbild einer Schaltungsanordnung zur Durchführung von Messungen in Verbindung mit der erfindungsgemäßen Sensorvorrichtung,
Figur 2 eine perspektivische Darstellung der Sensorvorrichtung nach Figur 1,
Figur 3 eine schematische Darstellung der Sensorvorrichtung nach Figur 1 gemäß einem ersten Grundlagenbeispiel,
Figur 4 eine schematische Darstellung der Sensorvorrichtung nach Figur 1 gemäß einem zweiten Grundlagenbeispiel,
Figur 5 eine schematische Darstellung der Sensorvorrichtung nach Figur 1 gemäß einem dritten Grundlagenbeispiel,
Figur 6 (Figuren 6A, 6B, 6C, und 6D) eine schematische Darstellung einer Sensorvorrichtung mit einer Mehrzahl von einzelnen Sensorelementen gemäß einem vierten Grundlagenbeispiel,
Figur 7 eine schematische Darstellung einer Abwandlung der Sensorvorrichtung gemäß Figur 6 mit einer Mehrzahl unterschiedlicher Sensorelemente als bevorzugtes Ausführungsführungsbeispiel der vorliegenden Erfindung.

### Beschreibung von Grundlagenbeispielen und einem bevorzugten Ausführungsbeispiel

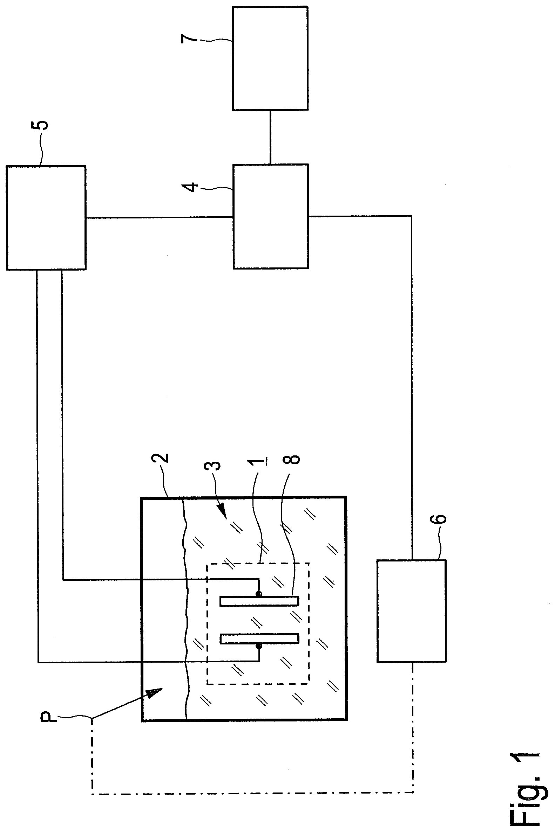

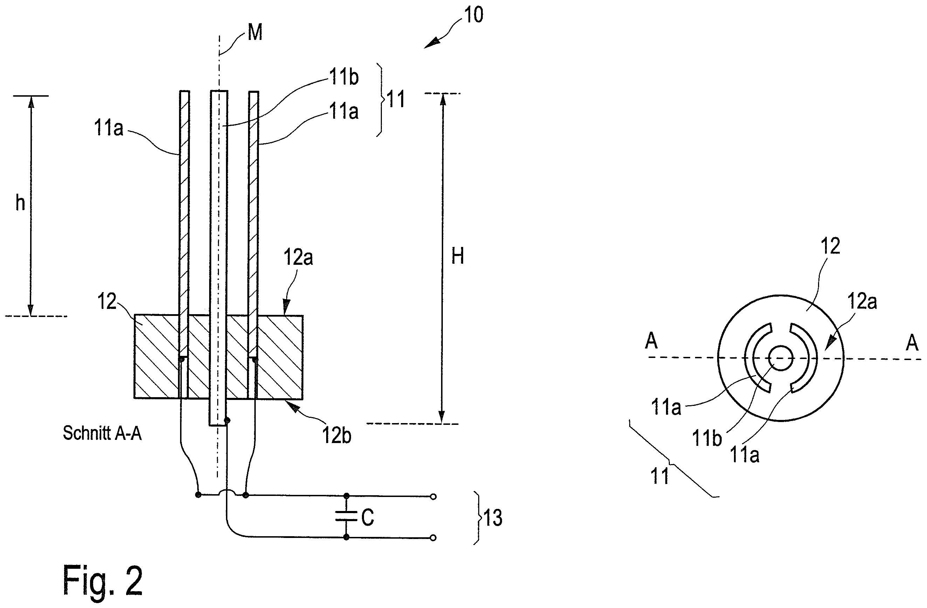

Nachstehend werden anhand des in Figur 1 gezeigten schematischen Blockschaltbilds der grundlegende Aufbau, die Wirkungsweise und die entsprechende Ansteuerung einer Sensorvorrichtung 1 gemäß der vorliegenden Erfindung beschrieben. Die Sensorvorrichtung 1 ist gemäß Figur 1 ist lediglich schematisch in vereinfachter Form dargestellt als ein kapazitiver Sensor mit zwei symbolischen Kondensatorplatten, während die weiteren Figuren 2 bis 4 konkrete Ausgestaltungen der Sensorvorrichtung 1 zeigen. Diese werden nachstehend noch beschrieben.

Figur 1 zeigt zur Veranschaulichung des Einsatzes der Sensorvorrichtung 1 zur Erfassung von Eigenschaften eines flüssigen Mediums, wie beispielsweise einer wässrigen Lösung, ein schematisiertes Blockschaltbild mit verschiedenen Komponenten, die zur Durchführung von Messungen geeignet sind und mittels denen die Sensorvorrichtung 1 angesteuert wird.

Im Einzelnen ist gemäß Figur 1 die Sensorvorrichtung 1 in einem Behälter 2 angeordnet, in welchem sich ein fluides Medium 3 befindet. Der Begriff des Behälters 2 ist in allgemeiner Form aufzufassen, wobei es sich um einen Tank in einem Gerät oder einer Maschine, eine Trommel einer Wascheinrichtung (Waschmaschine) oder dergleichen handeln kann. In jedem Fall beinhaltet der Behälter 2 das zu erfassende Medium 3, vorzugsweise eine wässrige Lösung, in einer Ausreichenden Menge, sodass mittels der Sensorvorrichtung 1 die Eigenschaften des Mediums 3 erfasst werden können. Zumindest kann mittels der Sensoreinrichtung 1 und ihrer entsprechenden Ansteuerung ein interessierender Parameter gemessen werden. Die Erfassung kann dabei kontinuierlich oder in vorbestimmten zeitlichen Abständen oder bei Bedarf erfolgen. Eine Steuerung mittels eines EDV-Programms ist möglich.

Die Sensorvorrichtung 1 ist zur Durchführung einer verlässlichen Messung ganz oder zumindest teilweise von dem Medium 3 umgeben. Die Sensorvorrichtung 1 ist in der nachfolgenden Beschreibung als Beispiel in einer Waschlauge (Medium 3) einer Wascheinrichtung (Waschmaschine) angeordnet. Mittels der Sensorvorrichtung 1 wird die Waschlauge hinsichtlich beispielsweise der Konzentration eines Waschmittels oder eines Verschmutzungsgrades überprüft. Die Erfindung ist hierauf jedoch nicht beschränkt, vielmehr können weitere Parameter der zu erfassenden wässrigen Lösung bestimmt werden. Beispielsweise können die Eigenschaften von Frischwasser (beispielsweise Schadstoffe oder Wasserhärte) bestimmt werden, oder es kann in Abhängigkeit von den erfassten Eigenschaften eines Frischwassers ein Waschvorgang in Verbindung mit einem Waschmittelverbrauch gesteuert oder geregelt werden.

Zur Durchführung einer entsprechenden Messung ist die Sensorvorrichtung 1 beispielsweise mit einer Steuerungseinrichtung 4 verbunden, die einerseits zur Steuerung der gesamten Abläufe der Erfassung der Eigenschaften des Mediums 3 dient und anderseits eine Ansteuerung der Sensorvorrichtung 1 veranlasst, sowie eine Auswertung der von der Sensorvorrichtung 1 abgegebenen Signale (Erfassungssignale) durchführt. Zu diesem Zweck ist die Steuerungseinrichtung 4 mit einem Ansteuerungsteil 5 verbunden, der in Abhängigkeit von Anweisungen und Befehlen der Steuerungseinrichtung 4 die Sensorvorrichtung 1 mit entsprechenden elektrischen Signalen ansteuert. Beispielsweise werden an die Sensorvorrichtung 1 (d. h. an die Sensorvorrichtungen gemäß den nachstehend beschriebenen Ausführungsbeispielen) zur Durchführung der entsprechenden Messungen elektrische Spannungen in Verbindung mit vorbestimmten Frequenzen oder Frequenzbereichen angelegt. Hierbei kann ein Verfahren in Verbindung mit der Impedanzspektroskopie zur Anwendung kommen.

Die Steuerungseinrichtung 4 ist auch vorgesehen zur Erfassung der Temperatur des Mediums 3 und kann hierzu mit einer in den Figuren nicht gezeigten und im Behälter 2 angeordneten Temperaturerfassungseinrichtung verbunden sein. Die Sensorvorrichtung 1 und der Ansteuerungsteil 5 können hinsichtlich ihrer Funktion auch zusammengefasst und in einer Einrichtung angegeben werden, wobei diese beispielsweise als eine Messeinrichtung bezeichnet werden kann. Die Steuerungseinrichtung 4 ist ferner mit einer Hauptsteuereinheit 6 verbunden, die direkt im Zusammenhang mit beispielsweise der Wascheinrichtung steht, in der der Behälter 2 angeordnet ist. In dem Fall, dass der Behälter 2 der Laugenbehälter einschließlich der Trommel der Wascheinrichtung ist, stellt die Hauptsteuereinheit 6 die eigentliche elektronische Steuerungseinheit der Wascheinrichtung dar.

In der Wascheinrichtung werden in der entsprechenden und programmierten Weise Wassermenge und Waschmittelzulauf sowie der Motorenbetrieb gesteuert. Die Steuerungseinrichtung 4 kann in Abhängigkeit von den Messergebnissen in Verbindung mit dem Betrieb der Sensorvorrichtung 1 die Hauptsteuereinheit 6 dahingehend beeinflussen, dass entsprechend der gewonnen Information und beispielsweise abweichend von einem vorbestimmten Programm eine größere oder kleinere Wassermenge oder eine größere oder kleinere Waschmittelmenge eingesetzt oder auch eine Veränderung der Waschtemperatur vorgenommen werden kann. Es kann mittels der Steuerungseinrichtung 4 in Verbindung mit der Hauptsteuereinheit 6 eine variable Steuerung oder auch eine Regelung im Gesamtsystem der Wascheinrichtung vorgenommen werden. Die allgemeine Einflussnahme der Hauptsteuereinheit 6 auf den Waschvorgang ist in Figur 1 symbolisch mittels einer gestrichelten Linie und einem Pfeil P in Richtung des Behälters 2 dargestellt.

Die Steuerungseinrichtung 4 kann ferner in Verbindung mit einer Speichereinrichtung 7 stehen, in der zur Durchführung der entsprechenden Messungen, d. h. zur Erfassung der Materialeigenschaften des Mediums 3 entsprechende Daten und Programme gespeichert werden können, auf die die Steuerungseinrichtung 4 bedarfsweise zugreifen kann uns wobei auch Erfassungsdaten oder verarbeitete Daten in der Speichereinrichtung 7 gespeichert werden können.

Bei der Durchführung von Messungen mittels der Sensorvorrichtung 1 können gemäß der vorstehenden Beschreibung der Sensorvorrichtung 1 auf eine entsprechende Anweisung der Steuerungseinrichtung 4 mittels des Ansteuerungsteils 5 vorbestimmte physikalische Größen, wie Strom, Spannung in Verbindung mit entsprechenden Frequenzen zugeführt werden. In Verbindung mit der durchgeführten Messung werden durch die Steuerungseinrichtung 4 über den Ansteuerungsteil 5 die Erfassungssignale der Sensorvorrichtung 1 aufgenommen und verarbeitet, gegebenenfalls in Verbindung mit aus der Speichereinrichtung 7 ausgelesenen Programmen oder Daten. Beispielsweise kann ein Vergleich mit Grunddaten hinsichtlich der Eigenschaften des Erfassten Mediums 3 durchgeführt werden, um absolute oder relative Änderungen zu erfassen, wobei auch auftretende Änderungen während vorbestimmter länger andauernder Vorgänge erfasst werden können.

In Abhängigkeit von dem Erfassungsergebnis, beispielsweise entsprechend einer Konzentration der erfassten Waschlauge in der Wascheinrichtung, kann die Hauptsteuereinheit 6 angewiesen werden, bestimmte Betriebsparameter zu ändern oder beizubehalten, und es können zum späteren Nachvollziehen von Messergebnissen und Abläufen der Wascheinrichtung die Messergebnisse in der Speichereinrichtung 7 gespeichert werden.

Während in der vorstehend beschriebenen Figur 1 die Anordnung der Sensorvorrichtung 1 innerhalb eines Messsystems oder einer Steuerungs- und Regelungssystems angegeben ist, zeigen die weiteren Figuren 2 bis 4 entsprechenden Ausführungsbeispiele der Sensorvorrichtung 1, die an die Stelle der Sensorvorrichtung 1 gemäß Figur 1 eingesetzt und im entsprechender Weise betrieben werden kann.

### Erstes Grundlagenbeispiel

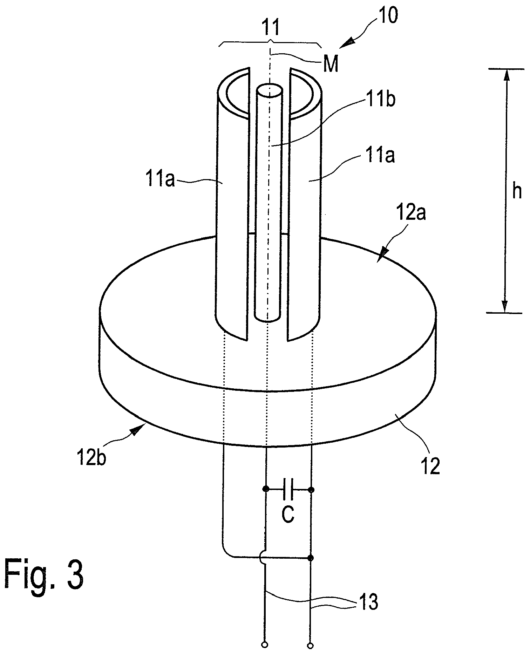

Die Figuren 2 und 3 zeigen ein erstes Grundlagenbeispiel der Sensorvorrichtung 10. Figur 2 zeigt eine Draufsicht auf die Anordnung der Sensorvorrichtung 10, sowie eine Schnittdarstellung entlang einer Schnittlinie A-A. Figur 3 zeigt eine perspektivische Darstellung der Sensorvorrichtung 10.

In der Darstellung gemäß Figur 1 ist die symbolische Sensorvorrichtung 1 als ein kapazitiver Sensor vereinfacht und schematisch durch zwei einander gegenüberliegende Kondensatorplatten 8 dargestellt. Die schematischen Kondensatorplatten veranschaulichen das Grundprinzip eines Plattenkondensators, bei dem zwischen den Platten ein Material mit vorbestimmten und/oder zu erfassenden dielektrischen Eigenschaften angeordnet ist. Im vorliegenden Fall gemäß Figur 1 und in Verbindung mit den Betrachtungen bei einer Wascheinrichtung wird das dielektrische Material durch das Medium 3, und beispielsweise durch eine wässrige Lösung wie eine Waschlauge gebildet.

Die Sensorvorrichtung 10 des ersten Ausführungsbeispiels umfasst gemäß der Darstellung in den Figuren 2 und 3 eine Sensoranordnung, bei der Elektroden (anstelle der schematischen Kondensatorplatten 8 gemäß Figur 1) in Form der einzelnen Elektroden 11 einander gegenüberliegend angeordnet sind. Insbesondere sind die Elektroden 11 der Sensorvorrichtung 10 gemäß dem ersten Grundlagenbeispiel derart angeordnet, dass zumindest eine äußere (erste) Elektrode 11a und im konkreten Fall der Figuren 2 und 3 zwei äußere Elektroden 11a, die als ein Teil einer Mantelfläche eines Hohlzylinders ausgebildet sind, einer im wesentlichen im Mittelpunkt der Grundfläche des Hohlzylinders angeordneten inneren (zweiten) Elektrode 11b gegenüberstehen. Jede der äußeren Elektroden 11a ist kleiner als die Hälfte der Mantelfläche des Hohlzylinders, so dass zwei voneinander getrennte und beabstandete Elektroden in einer Anordnung ähnlich der von Halbschalen gebildet werden, zwischen denen ein Zwischenraum in vorbestimmter Weise angeordnet ist. Die innere Elektrode 11b verläuft bzw. erstreckt sich entlang der Hauptachse oder Symmetrieachse des gedachten Hohlzylinders, der durch die beiden äußeren Elektroden 11a aufgespannt wird. Sämtliche Elektroden 11 sind in einer aus isolierendem Material bestehenden Grundplatte 12 angeordnet und mechanisch stabil befestigt, sodass ein dielektrisches Material und insbesondere das zu erfassende Medium 3 zwischen die äußeren Elektroden 11a und die innere Elektrode 11b gelangen kann, die sich im Wesentlichen senkrecht von der Grundplatte 12 weg erstrecken. Es werden somit die erste und die zweite Elektrode 11a und 11b von dem Medium 3 umströmt.

Die beiden äußeren Elektroden 11a sind zueinander in vorbestimmter und bevorzugt konstanter Weise beabstandet, sodass zwischen einer der äußeren Elektroden 11a und der inneren Elektroden 11b ein annähernd konstanter Abstand gebildet wird, und die jeweilige Länge der Elektroden 11 außerhalb der Grundplatte 12 etwa gleich ist. Sämtliche Elektroden 11 verlaufen somit entlang der Mittelachse des gedachten Holzylinders auf einer der Seiten der Grundplatte 12, gemäß den Darstellungen in den Figuren 2 und 3 auf der oberen Seite der Grundplatte 12. Die Seite der Grundplatte 12, auf der die Elektroden 11 angeordnet sind, wird als erste Seite oder als erste Oberfläche 12a bezeichnet.

Auf der anderen Seite der Grundplatte 12, d. h. auf der zweiten Seite oder Oberfläche 12b sind die jeweiligen Elektroden 11 mit elektrischen Anschlussleitungen 13 verbunden. Hierbei stellt die zumindest eine äußere Elektrode 11a den einen Pol dar. Im Falle der beiden äußeren (ersten) Elektroden 11a sind diese elektrisch miteinander verbunden und stellen denselben Pol der Elektrodenanordnung gemäß den Figuren 2 und 3 dar. Die innere (zweite) Elektrode 11b stellt den anderen Pol dar und ist ebenfalls mit einer der Anschlussleitungen 13 verbunden.

Figur 2 zeigt schematisch die Verbindung der entsprechenden Anschlussleitungen 13 mit den jeweiligen Elektroden 11. Hierbei ist zwischen dem einen Pol der äußeren Elektroden 11a und dem anderen Pol der inneren Elektrode 11b ein optionaler Kondensator C angegeben. Der Kondensator C dient zur Festlegung des Frequenzbereichs der Ansteuerungssignale, mittels denen die Sensorvorrichtung 10 angesteuert wird. Mit dem vorbestimmten Kapazitätswert des Kondensators C kann die Frequenz von bestimmten Messpunkten in einem Messablauf festgelegt werden. Die jeweiligen Anschlussleitungen 13 sind zur Durchführung entsprechender Messungen oder Erfassungen mit dem Ansteuerungsteil 5 (Figur 1) verbunden.

Gemäß den Figuren 2 und 3 ist die Grundplatte 12 als eine annähernd kreisförmige Scheibe mit einer vorbestimmten Dicke dargestellt, in deren Mitte die innere Elektrode 11b und um die innere Elektrode 11b mit einem vorbestimmten Abstand die zumindest eine und vorzugsweise beiden äußeren Elektroden 11a angeordnet sind. Die Ausführung der Grundplatte 12 als eine kreisförmige Scheibe stellt lediglich ein Beispiel dar, und die vorliegende Erfindung ist nicht auf diese Grundform festgelegt. Vielmehr können beliebige Formen der Grundplatte 12 auch mit einer unterschiedlichen Dicke vorgesehen sein, wobei auch die Anordnung der Elektroden 11 nicht notwendigerweise in der Mitte einer symmetrisch ausgebildeten Grundplatte 12 sein muss. In jedem Fall wird die Grundplatte 12 in eine entsprechende Öffnung in dem Behälter 2 eingesetzt, wobei entsprechende Abdichtungsmaßnahmen im Bereich der Öffnung vorgesehen sind zur Vermeidung eines unerwünschten Austretens des Mediums 3 aus dem Behälter 2 an der Einsetzstelle der Sensorvorrichtung 10. Die Sensorvorrichtung 10 befindet sich vorzugsweise im unteren Teil des Behälters 2 und weiter bevorzugt an einer tiefsten Stelle des Behälters 2, sodass auch bei kleinen Mengen des Mediums 3 eine zuverlässige Erfassung der Eigenschaften des Mediums 3 gewährleistet ist.

Die Eigenschaften des zu erfassenden Mediums betreffen beispielsweise eine Konzentration an Mineralien, an waschaktiven Substanzen sowie eine in Folge des Waschvorgangs aufgetretene und auch zunehmende Verschmutzung der Waschlauge. Es kann zumindest ein Parameter wie eine Konzentration bestimmter Substanzen ermittelt werden. Des Weiteren kann an der Sensorvorrichtung 10, insbesondere im Bereich der Grundplatte 12 eine Temperaturerfassungseinrichtung vorgesehen sein.

In den Figuren 2 und 3 ist bei der Darstellung der Sensorvorrichtung 10 eine erste Höhe h und eine zweite Höhe H angegeben. Die erste Höhe h betrifft die freie Länge der Elektroden 11 außerhalb der Grundplatte 12 im zu erfassenden Bereich des Behälters 2. Dieser Bereich der Elektroden ist damit vom Medium 3 umströmt.

Die zweite Höhe H stellt die Gesamtlänge der längsten Elektrode 11, beispielsweise der inneren Elektrode 11b dar, wobei ein Teil der inneren Elektrode 11b oberhalb und ein weiterer Teil unterhalb der Grundplatte 12 angeordnet ist. Der Teil unterhalb der Grundplatte 12 erstreckt sich somit weg von der zweiten Oberfläche 12b. Mit dem unterhalb der Grundplatte 12 liegenden Teil sind die Anschlussleitungen 13 und der Kondensator C verbunden.

Gemäß der Darstellung in den Figuren 2 und 3 besteht die äußere Elektrode 11a aus zwei Teilen, die entsprechend ihrer Form als Teil einer Hohlzylinderwand konzentrisch um die innere Elektrode 11b angeordnet sind. Die vorliegende Erfindung ist hierauf jedoch nicht festgelegt, und es besteht ebenfalls die Möglichkeit, die konzentrisch um die innere Elektrode 11b angeordneten äußeren Elektroden 11 a in Form von drei oder mehr etwa gleichartigen Teilen vorzusehen, die jeweils in einer Richtung senkrecht zu einer Grundplatte 12 entlang der Mittellinie M entsprechend zueinander beabstandet sind, sodass eine ungehinderte Strömung des Mediums 3 (Fluide) zwischen den einzelnen Elektrodenteilen auch bei im Verlauf des Waschvorgangs entstandenen Schwebstoffen gewährleistet ist. Sind beispielsweise zwei Paare von äußeren Elektroden 11a vorgesehen, so können zwei der vier größeren Elektroden 11a isoliert (d. h. mit einer Isolierschicht versehen) und die beiden anderen in einer nicht-isolierten Weise gegenüber dem Medium 3 im Behälter 2 ausgeführt sein. Sämtliche Teile der ersten bzw. äußeren Elektrode 11a stellen denselben Pol dar.

Die zumindest eine erste (äußere) Elektrode 11a kann alternativ auch einstückig als vollständiger Hohlzylinder ausgebildet werden, in dessen Innenraum die zweite (innere) Elektrode 11b im Wesentlichen entlang einer Symmetrieachse oder Mittellinie (M gemäß Figur 3) verläuft. Die erste Elektrode 11a in der Form eines Hohlzylinders weist einige Öffnungen in ihrer Zylinderwand auf, so dass das zu erfassende Medium 3 die beiden Elektroden 11 (11a und 11b) umströmen kann und insbesondere in den Raum zwischen den Elektroden 11 gelangen kann. Abweichend von der Darstellung der Draufsicht in Figur 2 ergibt sich dann ein vollständiger Hohlzylinder mit entsprechenden Strömungsöffnungen, mit denen eine stetige Umströmung des Bereichs zwischen den Elektroden 11 gewährleistet ist.

### Zweites Grundlagenbeispiel

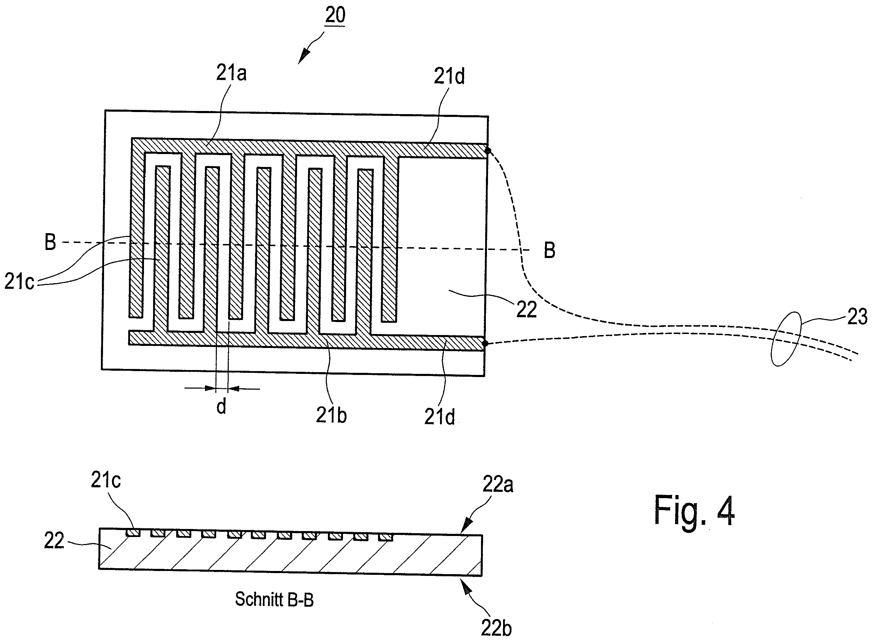

Figur 4 zeigt ein zweites Grundlagenbeispiel der Sensorvorrichtung 20, die als Sensorvorrichtung 1 in allgmeiner Weise in Figur 1 gezeigt ist. Figur 4 zeigt eine Draufsicht auf die Anordnung der Sensorvorrichtung 20, sowie eine Schnittdarstellung entlang einer Schnittlinie B-B.

Die Sensorvorrichtung 20 gemäß dem zweiten Grundlagenbeispiel umfasst erste und zweite Elektroden 21a und 21b, die auf einer gemeinsamen Grundplatte 22 angeordnet sind. Gemäß der Darstellung in Figur 4 ist in schematischer Weise die aus einem isolierenden Material bestehende Grundplatte 22 als eine rechteckförmige Platte angeordnet. Die Erfindung ist hierauf jedoch nicht festgelegt, vielmehr kann die Grundplatte 22 auch eine davon abweichende Form aufweisen.

Die erste und zweite Elektrode 21a und 21b sind in der Weise flächig auf der Grundplatte 22 angeordnet, dass diese von einer Seite ausgehend einzelne Elektrodenarme 21c aufweisen, die auf der Grundplatte 22 abwechselnd angeordnet sind. Die erste und zweite Elektrode 21a und 21b greifen kammförmig ineinander, wobei jedoch sämtliche Teile der beiden Elektroden 21a und 21b voneinander um einen vorbestimmten Abstand d beabstandet sind. Auf der aus isolierendem Material bestehenden Grundplatte 22 sind somit die beiden Elektroden 21a und 21b elektrisch von einander getrennt und jeweils elektrischen einem Pol zugeordnet. Die beiden Elektroden 21a und 21b bilden einen kapazitiven Sensor und sind auf einer ersten Seite oder ersten Oberfläche 22a der Grundplatte 22 angeordnet. Eine gegenüber liegende Oberfläche wird als zweite Oberfläche 22b bezeichnet.

Die beiden Elektroden 21a und 21b weisen jeweilige Anschlussbereiche 21 d auf, mit denen entsprechende Anschlussleitungen 23 verbunden sind. Die Anschlussleitungen 23 können auf der ersten oder der zweiten Oberfläche 22a oder 22b angeordnet sein. In gleicherweise wie beim ersten Grundlagenbeispiel kann ein Kondensator C zwischen die erste Elektrode 21a und die zweite Elektrode 21b und somit zwischen den elektrischen Polen angeordnet sein.

Figur 4 zeigt des Weiteren eine Schnittansicht der Sensorvorrichtung 20 gemäß dem zweiten Grundlagenbeispiel entlang der Schnittlinie B-B. Hieraus ist erkennbar, dass die einzelnen Teile der beiden Elektroden 21a und 21b versenkt und damit bündig mit der ersten Oberfläche 22a in der isolierenden Grundplatte 22 angeordnet sind. Die vorliegende Erfindung ist hierauf jedoch nicht festgelegt, und es können die Elektrodenteile der beiden Elektroden 21a und 21b auch teilweise in der Grundplatte 22 versenkt sein, oder können auf der Grundplatte 22 und insbesondere auf der ersten Oberfläche 22a derselben angeordnet sein. In jedem Fall sind sämtliche Teile der ersten Elektroden 21a um einen vorbestimmten Abstand d zur zweiten Elektrode 21b beabstandet. Der Bereich zwischen den Elektroden 21a und 21b und somit auch zwischen den Elektrodenarmen 22c verläuft auf der ersten Oberfläche 22a der Grundplatte 22 mäanderförmig.

### Drittes Gundlagenbeispiel

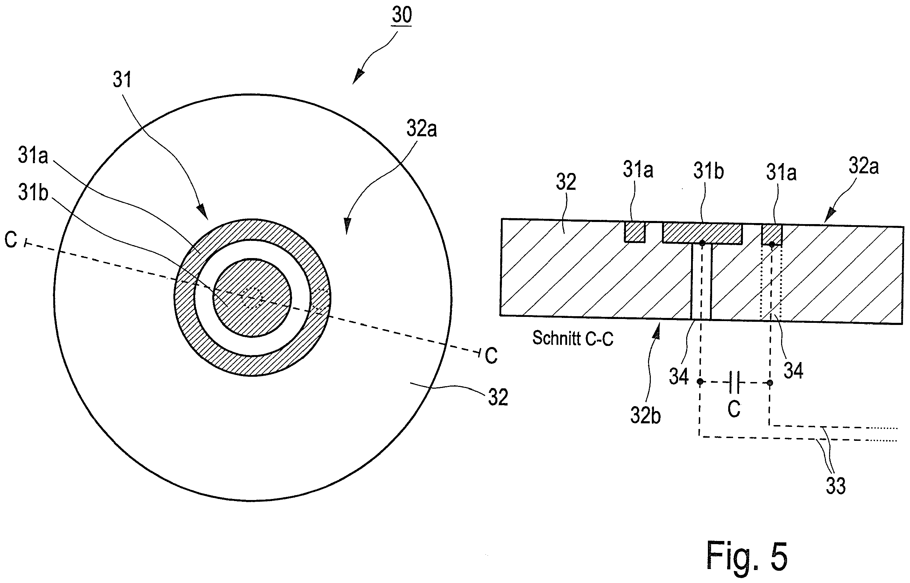

Ein drittes Grundlagenbeispiel der Sensorvorrichtung 30 wird nachstehend in Verbindung mit Figur 5 beschrieben.

Die in Figur 5 dargestellte Sensorvorrichtung 30 ist in gleicher Weise wie die Sensorvorrichtungen (10 und 20) gemäß dem ersten und zweiten Grundlagenbeispiel als kapazitiver Sensor ausgebildet. Die Sensorvorrichtung 30 gemäß dem dritten Grundlagenbeispiel umfasst Elektroden 31, bestehend aus einer äußeren Elektrode 31a und einer inneren Elektrode 31b. Die innere Elektrode 31b ist bevorzugt als eine Kreisfläche mit einem vorbestimmten Durchmesser aufgebaut, während die äußere Elektrode 31a kreisringförmig um die innere Elektrode 31b und beabstandet zu dieser angeordnet ist. Beide Elektroden 31 sind auf einer Grundplatte 32 aus einem isolierenden Material und dort auf einer ersten Oberfläche 32a der Grundplatte 32 angeordnet und befestigt und elektrisch voneinander isoliert. Der inneren und äußeren Elektrode 31a und 31b ist jeweils elektrisch ein Pol zugeordnet.

Die Grundplatte 32 ist gemäß dem vorliegenden dritten Grundlagenbeispiel nach Figur 5 als im Wesentlichen kreisförmige Scheibe mit einem vorbestimmten Durchmesser ausgebildet, der größer ist als der äußere
Durchmesser der kreisringförmigen äußeren Elektrode 31a. Es können die Elektroden 31 bündig mit der ersten Oberfläche 32a der Grundplatte 32 angeordnet und somit in diese eingesetzt sein, oder es können die Elektroden 31 ganz oder teilweise außerhalb des Materials der Grundplatte 32 auf der ersten Oberfläche 32a angeordnet sein. Der erste Fall ist in der neben der Draufsicht angeordneten Schnittdarstellung entsprechend der Schnittlinie C-C angegeben.

Die auf der Grundplatte 32 angeordneten Elektroden 31 sind mit entsprechenden Anschlussleitungen 33 verbunden. Hierzu weist die Grundplatte 32 im Bereich jeder der beiden Elektroden 31 und deren Anschlüsse an die Anschlussleitungen 33 entsprechende Öffnungen 34 in einer der ersten Oberfläche 32a gegenüber liegenden zweiten Oberfläche 32b auf, in denen die Anschlussleitungen 33 angeordnet sind. Die Anschlussleitungen 33 werden zur zweiten Oberfläche 32b der Grundplatte 32 und nach außen geführt. Hierbei werden die Anschlussleitungen weiter zu dem in Figur 1 gezeigten Ansteuerungsteil 5 geführt, mittels dessen entsprechende Ströme und Spannungen mit vorbestimmten Frequenzen an die Elektroden 31 angelegt werden. Ein Kondensator C kann zwischen die Elektroden 31 und damit zwischen die unterschiedlichen elektrischen Pole geschaltet werden.

Die Grundplatte 32 besteht aus einem isolierenden und festen Material, wobei die scheibenförmige Ausbildung lediglich beispielhaft ist. Die Erfindung ist hierauf nicht festgelegt. Vielmehr kann auch eine beliebige andere flächige Form für die Grundplatte 32 gewählt werden. In jedem Fall ist eine ausreichend ebene Fläche (erste Oberfläche 32a) zur Ausbildung der Elektroden 31 vorzusehen.

In Figur 5 sind im linken Teil in der Draufsicht die beiden Elektroden 31a und 31b als kreisringförmige oder scheibenförmige Anordnung dargestellt. Die Erfindung ist hierauf jedoch nicht festgelegt, und es kann beispielsweise die innere Elektrode 31b auch als ein Quadrat, Dreieck oder Rechteck ausgebildet sein, wobei in diesem Fall die äußere als eine Ringstruktur ausgebildete Elektrode 31a der Kontur der inneren Elektrode 31b folgt und um einen vorbestimmten Abstand zu dieser beabstandet ist. Mit der isolierenden Grundplatte 32 sind somit die beiden Elektroden 31a und 31b voneinander elektrisch isoliert.

Wird die Sensorvorrichtung 30 gemäß dem dritten Grundlagenbeispiel in einem Behälter wie dem Behälter 2 und vorzugsweise in einer Seitenwand im Bodenbereich oder einer Bodenwand des Behälters 2 angeordnet, dann wird die Grundplatte 32 gegenüber der Behälterwand abgedichtet, sodass kein Medium 3 im Bereich der eingesetzten Grundplatte 32 der Sensorvorrichtung 30 unerwünscht austritt.

### Abwandlungen des ersten bis dritten Grundlagenbeispiels

Hinsichtlich der vorstehend beschriebenen Grundlagenbeispiele 1 bis 3 ist jeweils eine Grundplatte 12, 22 oder 32 vorgesehen, die aus einem festen isolierenden Material besteht. Hierzu eigenen sich verschiedene Kunststoffe, Glas oder Glaskeramik. Das Grundmaterial der Grundplatte 12, 22 und 32 sowie auch der Grundplatten der nachfolgend beschriebenen Ausführungsbeispiele muss eine hohe Wasserbeständigkeit besitzen, wobei möglichst wenig Wasser durch das Material selbst aufgenommen wird. Zur Verminderung des Eindringens von Wasser oder weiteren Komponenten des Mediums 3 im Behälter 2 wird das Grundmaterial, d. h. die Oberflächen der Grundplatte geglättet und poliert. Ferner muss das Grundmaterial der Grundplatte 12, 22 und 32 möglichst stabile Eigenschaften in elektrischer Hinsicht aufweisen, wie beispielsweise die Isolationseigenschaften und eine möglichst stabile Dielektrizitätszahl. Des Weiteren ist es erforderlich, dass das Grundmaterial der Grundplatte 12, 22 und 32 eine vorbestimmte Festigkeit aufweist, sodass die Ausbildung einer Grundplatte in der vorstehend beschriebenen Form gewährleistet, dass diese mechanisch sicher in einer Öffnung des Behälters 2 in der Behälterwand vorzugsweise nahe dem Behälterboden oder im Behälterboden selbst eingebaut werden kann und langfristig und stabil eine Abdichtung gewährleistet ist. Dies ist insbesondere im Hinblick auf den Betrieb einer Wascheinrichtung zu berücksichtigen, bei der in Verbindung mit entsprechend hohen Schleuderdrehzahlen der Waschtrommel starke Vibrationen auftreten können.

Die in den Figuren abgebildeten Elektroden 11, 21 oder 31 können im Fall des ersten Grundlagenbeispiels aus Edelstahl gebildet werden, wobei nach einer entsprechenden Vorbehandlung die beiden äußeren Elektroden 11a und die innere Elektrode 11b mit einem elektrochemisch inerten Material, wie beispielsweise Gold, beschichtet werden. Die Beschichtung kann beispielsweise mittels Sputtern erfolgen. Hierbei können die Elektroden ganz oder zumindest teilweise mit dem inerten Material (wie z. B. Gold) beschichtet werden.

Es ist erforderlich, dass alle Oberfläche der Elektroden 11, 21 oder 31, im Falle des ersten Grundlagenbeispiels sämtliche mit dem Medium 3 im Behälter 2 in Berührung kommende Flächen, so glatt wie möglich ausgeführt sind, um jegliches Eindringen auch geringer Mengen von Wasser oder weiterer Substanzen des Mediums 3 in das Material der Elektroden 11, 21 oder 31 zu vermeiden.

Das Grundmaterial der Elektroden 11, 21 oder 31 in sämtlichen Gundlagenbeispielen kann beispielsweise auch in Form einer Keramik oder Glaskeramik bereit gestellt werden, wobei die Oberfläche der aus diesen Werkstoffen gebildeten Teile und Komponenten (Elektroden und/oder Grundplatte) geschliffen und auf Hochglanz poliert wird, und es kann nach einer entsprechenden Behandlung der polierten Oberflächen eine Schicht mit einem inerten und elektrisch leitenden Material, z. B. eine Goldschicht, aufgetragen werden. Dies kann beispielsweise mittels Sputtern erfolgen.

Mit dem entsprechenden Gestalten der Oberflächen beispielsweise der Elektroden 11, 21 und 31 (und auch der Grundplatte 12, 22 und 32) kann das Eindringen von Wasser oder weiteren Inhaltsstoffen des Mediums 3 vermieden werden, so dass eine ungünstige Beeinflussung von Messergebnissen durch eine Veränderung der elektrischen und auch der mechanischen Eigenschaften der Elektroden 11, 21 oder 31 und auch der Grundplatte 12, 22 oder 32 vermieden werden kann.

Im Falle des ersten Grundlagenbeispiels (Figuren 2 und 3) können die Elektroden 11a und 11b aus einer Keramik oder Glaskeramik in der entsprechenden halbschalenförmigen Ausgestaltung (äußere Elektroden 11a) oder stabförmig (innere Elektrode 11b) gebildet werden. Nach jeweiligen vorbereitenden Maßnahmen erfolgt die Beschichtung mit dem inerten leitfähigen Material, z. B. Gold, und bedarfsweise ein weiteres Polieren. Bei einer einteiligen Ausführung der Elektroden 11 des ersten Ausführungsbeispiels kann die zumindest eine äußere Elektrode sowohl auf ihrer äußeren als auch ihrer inneren (d. h. auf den dem Medium ausgesetzten Oberflächen) mit dem inerten Material beschichtet werden. Zumindest ist die innere Oberfläche der zumindest einen äußeren Elektrode 11a, die der inneren Elektrode 11b gegenüber liegt, mit dem inerten Material zu beschichten und mittels der Anschlussleitungen galvanisch zu verbinden. Dies gilt auch bei der alternativen Ausführung der äußeren Elektrode des ersten Grundlagenbeispiels in Form eines (mit Öffnungen versehenen) vollständigen Hohlzylinders, wobei hier zumindest die Innenseite (gegenüber der inneren Elektrode 11b) beschichtet ist. Die Anschlussleitungen 13 werden im beschichteten Bereich mit den jeweiligen Elektroden 11a und 11b verbunden, wobei bei mehrteiligen äußeren Elektroden 11a sämtliche Teile der äußeren Elektroden 11a mit demselben Anschluss (Pol) verbunden werden.

Im Falle der flächig ausgebildeten Elektroden gemäß den Sensorvorrichtungen 20 und 30 des zweiten und dritten Grundlagenbeispiels besteht die Möglichkeit, nach einer entsprechenden Vorbehandlung der Oberfläche (zumindest der jeweiligen ersten Oberfläche 22a und 32a) der jeweiligen Grundplatte 22 und 32 das Muster und die Ausgestaltung der jeweiligen Elektroden 21 und 31 mittels eines elektrochemisch inerten Materials wie beispielsweise Gold zu bilden. Hierbei kommt ebenfalls die Möglichkeit des Sputterns in Betracht. Auch können die Elektroden 21 und 31 des zweiten und dritten Ausführungsbeispiels aus einem in der Grundplatte 22 und 23 ganz oder teilweise versenkten metallischen Leiter gebildet werden, der mit einem inerten leitenden Material (z. B. Gold) beschichtet sein kann.

In den Figuren sind die jeweiligen Elektroden 11, 21 und 31 mittels entsprechender Anschlussleitungen 13, 23 oder 33 verbunden, wobei diese ihrerseits wieder mit dem in Figur 1 gezeigten Ansteuerungsteil 5 verbunden sind. Da die Elektroden 11,21 oder 31 gemäß der vorstehenden Beschreibung bevorzugt aus einem elektrochemisch inerten Material bestehen oder zumindest mit diesen beschichtet sind (wie beispielsweise Gold) ist es sinnvoll, keine weiteren unterschiedlichen Metalle innerhalb der jeweiligen Sensorvorrichtung 10, 20 oder 30 zu verwenden. Bevorzugt werden daher die zumindest mit Gold beschichteten Elektroden mit Golddrähten kontaktiert zur Bildung der erforderlichen Anschlussleitungen 13, 23 und 33. Es kann die Kontaktierung durch Bonden oder Löten erfolgen, wobei auch ein leitfähiger Klebstoff verwendet werden kann.

Grundsätzlich befinden sich die Anschlussleitungen 13, 23 und 33 der jeweiligen Elektroden nicht im Bereich des Mediums 3, werden somit nicht von dem Medium 3 umströmt, da hierdurch eine Beeinflussung des Mediums oder eine Beeinflussung der Anschlussleitungen und der Kontaktpunkte sowie der elektrischen Eigenschaften der gesamten Sensoranordnung auftreten kann. Vielmehr sind die Anschlussleitungen 13, 23 und 33 in entsprechende Vertiefungen oder Öffnungen der jeweiligen Grundplatte 12, 22 oder 32 angeordnet und werden auf der Seite der jeweiligen Grundplatte 12, 22 oder 32 (d. h. ausgehend von der zweiten Oberfläche 12a, 22a, 32a) nach Außen geführt, die der mit dem Medium 3 beaufschlagten Seite (der zweiten Oberfläche 12b, 22b, 32b) gegenüber liegt. Falls erforderlich werden auch hier entsprechende Abdichtungsmaßnahmen der Anschlussleitungen 13, 23 und 33 durch temperaturfeste und auch gegenüber dem Medium 3 beständige Materialien wie beispielsweise Kunststoffe vorgenommen.

Für die jeweils mehrteilige Elektrodenanordnung der vorstehend beschriebenen Sensorvorrichtungen 10, 20 und 30 dient somit die jeweilige Grundplatte 12, 22 und 32 als eine Trägerplatte oder ein gemeinsamer Träger. Die Grundplatte 12, 22 oder 32 ist in der vorstehenden Beschreibung der Ausführungsbeispiele einstückig dargestellt. Sie kann jedoch auch aus mehreren miteinander verbundenen und abgedichteten Teilen oder aus verschiedenen Schichten bestehen, oder kann auch aus mehreren der genannten Materialien bestehen, wobei verschiedene Teile aus unterschiedlichen Materialien bestehen. In gleicher Weise wie bei den Elektroden der Sensorvorrichtung 1 können Materialien der Grundplatte 12, 22 und 32 aus Kunststoffen, Keramik oder Glaskeramik ausgewählt werden. Durch eine entsprechende Behandlung der jeweiligen Oberflächen durch Glätten und Polieren kann das Eindringen von Fremdstoffen in das Material der Grundplatte 12, 22 und 32 vermieden werden. Dies betrifft insbesondere die dem Medium 3 ausgesetzten Oberflächen. Die Materialien können auch miteinander verbunden werden, so dass beispielsweise eine Grundplatte aus mehreren Schichten unterschiedlichen Materials gebildet werden kann.

Bei der Anwendung der verschiedenen Grundlagenbeispiele der Sensorvorrichtung gemäß der vorstehenden Beschreibung besteht des weiteren die Möglichkeit, auch beispielsweise Frischwasser in Wasseraufbereitungsanlagen, oder auch Abwässer von Haushalten oder Industrie auf einen Schadstoffgehalt zu untersuchen oder auch erwünschte oder unerwünschte Konzentrationsänderungen von Substanzen im Wasser zu erfassen. Dies kann für eine Wasserversorgung oder im Hinblick auf Reinigungsvorgänge wässriger Lösungen sinnvoll sein. Mit den erfassten Werten kann eine Steuerung oder Regelung durchgeführt werden. Auch können Kühlmedien auf der Basis wässriger Lösungen im Hinblick auf die Konzentration von Zusatzstoffen in der Lösung überprüft werden.

Die jeweiligen Sensorvorrichtungen sind derart in dem Behälter 2, und beispielsweise innerhalb einer Wascheinrichtung, angeordnet, dass diese vom Medium 3 umströmt werden und in Folge der entsprechenden Strömung unerwünschte Ablagerungen an den Sensorvorrichtungen vermieden werden. In Figur 1 zeigt der Pfeil P die Möglichkeit der Beeinflussung des Waschvorgangs der Wascheinrichtung in dem Behälter 2 mit in Verbindung mit dem Medium 3 durch die Hauptsteuereinheit 6, die sowohl mit einem internen Programm mit vom Benutzer eingestellten Parametern als auch mit Erfassungsergebnissen der Steuerungseinrichtung 4 den Waschvorgang steuert oder regelt.

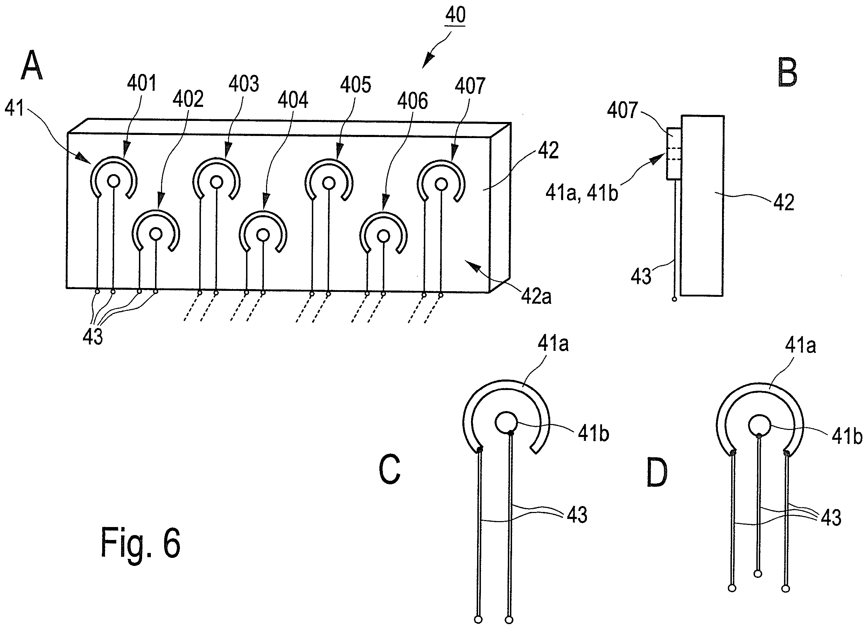

Nachstehend wird unter Bezugnahme auf Figur 6 ein viertes Grundlagenbeispiel beschrieben. Die Sensorvorrichtung 40 ist beispielsweise in dem Behälter 2 gemäß Figur 1 angeordnet und dem zu erfassenden Medium 3 zumindest teilweise ausgesetzt.

Fig. 6 mit den Teilfiguren 6A, 6B, 6C und 6D zeigt eine Sensorvorrichtung 40 gemäß dem vierten Grundlagenbeispiel, wobei in ähnlicher Weise wie im dritten Grundlagenbeispiel Elektroden 41 auf einer Grundplatte 42 ausgebildet sind. Die Elektroden 41 bestehen jeweils aus einer äußeren Elektrode 41a sowie einer inneren Elektrode 41b. Diese Anordnung der Elektroden 41 (41a und 41b) bildet ein jeweiliges Sensorelement, wobei in Fig. 6A beispielsweise sieben Sensorelemente 401 bis 407 gezeigt sind. Die Sensorelemente 401 bis 407 sind zueinander in einer vorbestimmten räumlichen Lage angeordnet. Hierbei sind die Sensorelemente 401 bis 407 (ein erstes bis siebtes Sensorelement) in verteilter Weise auf der Grundplatte 42 angeordnet, die beispielsweise aus einem Kunststoff, aus Glas, aus Glaskeramik oder einer Keramik bestehen kann. Die einzelnen Elektroden 41a und 41b der jeweiligen Sensorelemente 401 bis 407 sind auf der entsprechend vorbereiteten Oberfläche einer Seite der Grundplatte 42 ausgebildet, wobei die Elektroden 41 vollständig auf dieser Oberfläche oder auch in der Oberfläche zumindest teilweise versenkt angeordnet werden können, so dass die Elektroden 41 der Sensorelemente 401 bis 407 teilweise aus der entsprechenden Oberfläche der Grundplatte 42 hervorstehen oder bündig mit dieser Oberfläche abschließen. Vorzugsweise ist die Grundplatte 42 eine ebene Platte mit einer vorbestimmten Dicke und einer für die Sensorelemente vorbereiteten Oberfläche.

Jedes Sensorelement 401 bis 407 umfasst Anschlussleitungen 43 mittels denen die einzelnen Elektroden 41a und 41b mit einem beispielsweise in Fig. 1 gezeigten Ansteuerungsteil 5 verbunden werden können. Hierbei kann die äußere Elektrode 41a, die entsprechend einer Teilringstruktur ausgeführt ist, mittels einer der Anschlussleitungen 43 verbunden werden, oder es können beide Enden der in einem vorbestimmten Bereich unterbrochenen äußeren Elektrode 41a mit Anschlussleitungen 43 verbunden sein. Die innere (mittlere) Elektrode 41b ist mit einer anderen der Anschlussleitungen 43 verbunden. Mittels des Ansteuerungsteils 5 (Fig. 1) können verschiedene Prüfsignale oder Ansteuerungssignale (Messsignale, beispielsweise Spannungen und Ströme als Gleichgrößen oder mit vorbestimmten Frequenzen) an die jeweiligen Elektroden 41 (41a, 41b) zur Durchführung einer Messung angelegt werden.

In der in Fig. 6A gezeigten Anordnung des vierten Grundlagenbeispiels sind sämtliche Sensorelemente 401 bis 407 mit Ausnahme entsprechender unvermeidbarer Toleranzen im Wesentlichen gleich ausgebildet. Hinsichtlich der Durchführung einer Messung ergeben sich somit etwa gleich große Kapazitätswerte der einzelnen Sensorelemente im unbelasteten Fall, d.h. ohne ein entsprechendes zu erfassendes Medium. Zur Messung wird die gesamte Sensorvorrichtung 40 mit dem zu erfassenden Medium 3 gemäß der Darstellung in Fig. 1 in Verbindung gebracht (d. h. vom zu erfassenden Medium umgeben bzw. umströmt), so dass mit sämtlichen Sensorelementen 401 bis 407 oder mit einer vorbestimmten Auswahl oder einzelnen Sensorelementen eine oder mehrere Messungen durchgeführt werden können.

Jedes einzelne Sensorelement 401 bis 407 stellt somit eine individuelle Teilkapazität oder einen Einzelsensor dar. Die Sensorelemente 401 bis 407 können in gleicher Weise gemeinsam (gleichzeitig) angesteuert werden, oder es können jeweils einzelne Sensorelemente gezielt und in entsprechender zeitlicher Reihenfolge zur Durchführung einer Messung durch den Ansteuerungsteil 5 und auch in unterschiedlicher Weise angesteuert werden. Hierbei können die einzelnen für eine Messung ausgewählten Sensorelemente zueinander in Reihe oder parallel geschaltet werden, so dass sich unterschiedliche Kapazitäten und Impedanzen ergeben. Mit der Möglichkeit einer Reihen- oder Parallelschaltung einzelner Sensorelemente können mit den sich ergebenden unterschiedlichen Impedanzen weite oder vorbestimmte kleinere Frequenzbereiche der Ansteuerungssignale verarbeitet werden. Es kann eine Anpassung an verschiedenen Messbereiche vorgenommen werden durch eine vorbestimmte Reihen- oder Parallelschaltung der an einer bestimmten Messung beteiligten Sensorelemente. Eine Anpassung an spezielle Eigenschaften eines zu erfassendes Mediums 3 oder an eine Messeinrichtung (beispielsweise Ansteuerungsteil 5 gemäß Fig. 1) sind ebenfalls auf einfache Weise möglich. Mittels der Ansteuerungseinrichtung 5 und/oder der Steuerungseinrichtung 4 kann die Art der Verbindung der einzelnen Sensorelemente bestimmt werden, wobei ebenfalls die Messung daran angepasst wird. Durch die Möglichkeit der Einstellung einer vorbestimmten Impedanz (Anpassung) könne elektronische Schaltungen zur Auswertung erfasster Signale einfacher ausgeführt werden.

Im Einzelnen können mittels des Ansteuerungsteils 5 die in Figur 6A gezeigten im Wesentlichen gleichartigen Sensorelemente 401 bis 407 einzeln unabhängig von jeweils anderen Sensorelementen angesteuert werden, beispielsweise mit unterschiedlichen elektrischen Werten, wie Spannungen und Frequenzen. Es können auf diese Weise unterschiedliche Kombinationen einer Reihen- oder Parallelschaltung der einzelnen Sensorelemente 401 bis 407 verwirklicht werden, wobei auch bestimmte (ausgewählte) Sensorelemente zueinander in Reihe und andere zueinander parallel geschaltet werden können.

Beispielhaft sind in Figur 6A sieben Sensorelemente 401 bis 407 der Sensorvorrichtung 40 gezeigt, wobei die Erfindung jedoch nicht auf diese Anzahl der Sensorelemente beschränkt ist. Vielmehr können mehr oder weniger Sensorelemente bedarfsabhängig vorgesehen sein.

Ungeachtet der im Wesentlichen gleichartigen mechanischen oder geometrischen Ausbildung der Sensorelemente 401 bis 407 auf der Grundplatte 42 können die Sensorelemente 401 bis 407 bzw. die zugehörigen Elektroden 41 (41a, 41b) aus einem gleichartigen leitenden Material bestehen, das auf die Grundplatte 42 aufgebracht ist. Einzelne der Sensorelemente 401 bis 407 oder Gruppen von Sensorelementen (eine Auswahl aus den Sensorelementen 401 bis 407) können aus unterschiedlichen Materialien bestehen. Als Materialien zur Ausbildung jeweiliger Sensorelemente 401 bis 407 und somit der jeweiligen Elektroden 41 kommen beispielsweise Gold, Platin und Chrom in Frage. Einzelne Sensorelemente 401 bis 407, bestehend aus unterschiedlichen Materialien, können in diesem Zusammenhang in Verbindung mit einer Messung auch jeweils unterschiedlich mittels des Ansteuerungsteils 5 gemäß Fig. 1 angesteuert werden. Eine jeweilige Auswertung der Ausgangssignale der jeweiligen Sensorelemente 401 bis 407 in Verbindung mit einer Messung kann auch davon abhängig gemacht werden, aus welchem Material das jeweilige Sensorelement 401 bis 407 besteht.

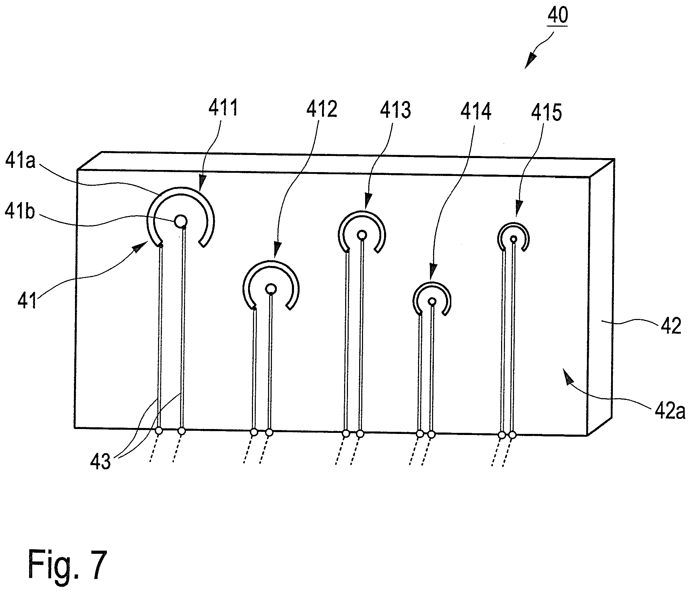

Figur 7 zeigt ein bevorzugtes Ausführungsbeispiel der vorliegenden Erfindung, das auf dem vierten Grundlagenbeispiel gemäß den Figuren 6A bis 6D aufbaut.

Somit zeigt Fig. 7 eine ähnliche Anordnung von einzelnen Sensorelementen, beispielsweise die Sensorelemente 411 bis 415, wie sie in Fig. 6A gezeigt ist. Es liegt ebenfalls eine Mehrzahl der Sensorelemente 411 bis 415 vor, wobei jedoch im Gegensatz zur Anordnung gemäß Fig. 6A die Sensorelemente der Sensorvorrichtung 40 jeweils eine unterschiedliche Größe (d. h. unterschiedliche Abmessungen) aufweisen. Die jeweilige Anordnung der Sensorelemente und insbesondere der Elektroden 41 (äußere Elektrode 41a und innere Elektrode 41b) weist bei den verschiedenen Sensorelementen 411 bis 415 unterschiedliche mechanische (geometrische) Abmessungen auf. Die Anordnung gemäß Fig. 7 des bevorzugten Ausführungsbeispiels umfasst somit die Sensorvorrichtung 40 mit beispielsweise den fünf Sensorelementen 411 bis 415, wobei sämtliche der Sensorelemente 411 bis 415 mit unterschiedlichen Abmessungen aus gleichen oder auch aus einem unterschiedlichen Material bestehen können. In gleicher Weise wie bei der Anordnung gemäß Fig. 6A können einzelne Sensorelemente oder sämtliche Sensorelemente 411 bis 415 oder Gruppen derselben mittels des Ansteuerungsteils 5 gemäß Fig. 1 in Verbindung mit einer durchzuführenden Messung angesteuert werden. Es können die einzelnen Sensorelemente 411 bis 415 auf der vorzugsweise ebenen Grundplatte 42 in ähnlicher Weise angeordnet werden, wie es in Fig. 6A gezeigt ist. Jedes Sensorelement 411 bis 415 weist die zur Ansteuerung erforderlichen Anschlussleitungen 43 auf.

Als Materialien für die einzelnen Elektroden der Sensorelemente 411 bis 415 kommen ebenfalls Gold, Platin, Chrom und dergleichen (Metallbeschichtungen) in Frage. Hierbei können die Beschichtungen zur Ausbildung der jeweiligen Elektroden beispielsweise mittels Sputtern auf die entsprechend vorbereitete Oberfläche der Grundplatte aufgebracht werden.

In Figur 6 sind auf der Grundplatte 42 beispielsweise sieben Sensorelemente 401 bis 407 gezeigt, und es sind beispielsweise 5 Sensorelemente 411 bis 415 auf der Grundplatte 42 gemäß Fig. 7 angeordnet. Die vorliegende Erfindung wird anhand dieser Darstellungen beschrieben. Sie ist jedoch nicht auf die Anzahl und die Anordnung der jeweiligen Sensorelemente 401 bis 415 festgelegt. Vielmehr kann eine größere oder kleinere Anzahl von Sensorelementen und in beliebiger Anordnung auf der jeweiligen Grundplatte 42 vorgesehen sein. In jedem Fall ist jedes der Sensorelemente über individuelle Anschlussleitungen 43 von außen zugänglich und damit ansteuerbar, so dass jedes Sensorelement 401 bis 415 getrennt oder in Gruppen angesteuert werden kann.

In gleicher Weise wie es in Fig. 6A gezeigt ist, stellen die einzelnen Sensorelemente 411 bis 415 gemäß Fig. 7 einzelnen Kapazitäten unterschiedlicher Art dar, wobei ferner auch unterschiedliche Materialien berücksichtigt werden können. Die Anordnung gemäß Fig. 7 mit den fünf Sensorelementen 411 bis 415 stellt somit ein 5-Bit-Sensor-Array dar. Die jeweiligen Sensorelemente 411 bis 415 werden dem zu erfassenden Medium 3 ausgesetzt, und es kann eine entsprechende Messung durch Bereitstellen elektrischer Signale (Spannungen, Ströme, Frequenzen) durchgeführt werden. Die Möglichkeiten einer vorbestimmten Reihen- oder Parallelschaltung mit einer Einstellung einer bestimmten Impedanz oder Kapazität bestehen in gleicher Weise, wie sie im Zusammenhang mit der Sensoranordnung gemäß Figur 6 beschrieben sind. Weitere Möglichkeiten zur Einstellung unterschiedlicher Kapazitäten und Impedanzen durch individuelle Ansteuerung der einzelnen Sensorelemente ergeben sich durch die unterschiedliche Größe der Sensorelemente des Ausführungsbeispiels gemäß Figur 7 und die unterschiedlichen elektrischen Eigenschaften.

Es können mit der Sensorvorrichtung in gleichartiger oder ähnlicher Ausführung neben den kapazitiven Messungen auch induktive und resistive Messverfahren oder eine Kombination derselben verwendet werden. Mit der Anordnung einer Sensorvorrichtung gemäß 6A und 7 mit einer Mehrzahl von einzelnen Sensorelementen gleicher oder unterschiedlicher Art wird ein Multisensor-Array gebildet, bei dem durch entsprechende Ansteuerung sämtlicher Sensorelemente 401 bis 415 oder einzelner oder eines Teils der Sensorelemente 401 bis 415 in selektiver Weise die Empfindlichkeit der Sensorvorrichtung 40 einem Messverfahren, einer Messanordnung und/oder einem zu erfassenden Medium 3 angepasst werden kann. Hierzu besteht die Möglichkeit, mittels der Anordnung einer Vielzahl einzelner Sensorelemente 401 bis 415 die Impedanz der jeweiligen Sensorvorrichtung 40 an gegebene Bedingungen oder eine Auswertungseinrichtung anzupassen, indem selektiv sämtliche oder einzelne Sensorelemente mit unterschiedlicher Größe und aus unterschiedlichen Materialien bestehend angesteuert werden können.

Bei der Anordnung des bevorzugten Ausführungsbeispiels gemäß Fig. 7 mit den Sensorelementen 411 bis 415 mit unterschiedlicher Größe können die Größenunterschiede jeweiliger Sensoren zueinander in einem bestimmten Verhältnis stehen. Beispielsweise kann die Fläche der einzelnen Sensorelemente derart bemessen sein, dass die zweitgrößte Fläche der Hälfte der drittgrößten Fläche, die drittgrößte Fläche der Hälfte der zweitgrößten Fläche, die viertgrößte Fläche der Hälfte der drittgrößten usw. entspricht. Auf diese Weise stehen die jeweiligen wirksamen Flächen der einzelnen Sensorelemente 411 bis 415 in einem vorbestimmten Verhältnis zueinander, so dass durch eine geeignete Reihen- oder Parallelschaltung der einzelnen Sensorelemente (wobei Teilsensoren gebildet werden) die Gesamtkapazität der Sensorvorrichtung 40 in Stufen eingestellt werden kann. Der Anzahl der Sensorelemente bzw. Teilsensoren entspricht der Anzahl von Bits. Sind beispielsweise 8 Sensorelemente (Teilsensoren) vorgesehen, entspricht dies einem 8-Bit-System, wobei in Verbindung mit einer entsprechenden Ansteuerung durch den Ansteuerungsteil 5 die Kapazität in 256 Stufen eingestellt werden kann.

Abweichend hiervon können auch andere Größenverhältnisse der einzelnen Sensorelemente zueinander auf der Grundplatte 42 ausgebildet werden. Hierbei können auch unterschiedliche Empfindlichkeiten der Sensorvorrichtung (Kapazitäten und Impedanzen) eingestellt werden.

Die Grundplatte 42 stellt eine Trägereinrichtung dar und besteht aus einem isolierenden Material, beispielsweise einem Kunststoff, einer Keramik, einer Glaskeramik oder Glas. Die einzelnen Elektroden 41 der Sensorelemente 401 bis 415 können mittels Sputtern aufgebracht werden. Es sind jedoch auch andere Verfahren, wie beispielsweise Kleben oder Galvanisieren, möglich, um die jeweiligen Materialien auf die Oberfläche der Grundplatte 42 aufzubringen.

Gemäß den Anordnungen in den Figuren 6A und 7 ist die Grundplatte 42 jeweils als ebene Fläche mit einer vorbestimmten Dicke entsprechend dem verwendeten Material und der erforderlichen Fläche (Anzahl der Sensorelemente) dargestellt. Die Erfindung ist jedoch hierauf nicht beschränkt. Vielmehr kann die Grundplatte auch eine gekrümmte oder sphärische Oberfläche einseitig oder beidseitig aufweisen, auf der die jeweiligen Sensorelemente 401 bis 415 angeordnet sein können. Die vorstehend beschriebenen Sensorelemente 401 bis 415 können lediglich auf einer der Oberflächen der Grundplatte 42 oder auf beiden Oberflächen (beidseitig) ausgebildet sein. Die Sensorvorrichtung 40 ist beispielsweise in dem Behälter 2 gemäß Fig. 1 angeordnet und zumindest teilweise von dem zu erfassenden Medium 3 umströmt.

## Patentansprüche

1. Sensorvorrichtung zur Erfassung von Eigenschaften fluider Medien in einem Behälter, mit
- zumindest einer aus einem isolierenden Material gebildeten Grundplatte (42) mit einer ersten den Medien (3) ausgesetzten Oberfläche (42a),
- mehr als zwei gleichartige Sensorelemente ( 411 bis 415) mit jeweils zumindest einer ersten und einer zweiten Elektrode (41a, 41b), die gegeneinander isoliert auf der ersten Oberfläche (42a) der Grundplatte angeordnet sind und von den Medien (3) umströmt werden,
- einer Ansteuerungseinrichtung (5) zur Ansteuerung der Sensorelemente, und
- wobei die mehreren Sensorelemente ( 411 bis 415) in einer vorbestimmten räumlichen Lage zueinander und auf der ersten Oberfläche (42a) verteilt angeordnet sind und jedes Sensorelement ( 411 bis 415) über individuelle Anschlussleitungen (43) ansteuerbar ist,
**dadurch gekennzeichnet, dass**
- die mehreren Sensorelemente ( 411 bis 415) in ihren geometrischen Abmessungen unterschiedlich ausgeführt sind, und wobei die Größe der unterschiedlich ausgeführten Sensorelemente (411 bis 415) in vorbestimmter Weise abgestuft ist, und
- die Ansteuerungseinrichtung (5) ausgebildet ist, sämtliche Sensorelemente oder Gruppen der Sensorelemente zur Erfassung der Eigenschaften der fluiden Medien (3) anzusteuern.

2. Sensorvorrichtung nach Anspruch 1, wobei die einzelnen Sensorelemente ( 411 bis 415) aus einem gleichen oder unterschiedlichen leitenden Material gebildet sind.

## Claims

1. Sensor device for detecting properties of fluid media in a container, comprising
- at least one base plate (42) made of an insulating material with a first surface (42a) exposed to the media (3),
- more than two sensor elements (411 to 415) of the same type each having at least a first and a second electrode (41a, 41b) which are arranged on the first surface (42a) of the base plate and being insulated from each other and around which the media flow,
- a drive device (5) for driving the sensor elements, and
- wherein the multiple sensor elements (411 to 415) are arranged in a predetermined spatial position relative to each other and spread over the first surface (42a), and each sensor element (411 to 415) being drivable via individual connection lines (43),
**characterized in that**
- the multiple sensor elements (411 to 415) are differently shaped in their geometrical dimensions, and wherein the size of the differently shaped sensor elements (411 to 415) is graded in a predetermined way, and
- the drive device (5) is configured to drive all sensor elements or groups of sensor elements for detecting the properties of the fluid media (3).

2. Sensor device according to claim 1, wherein the individual sensor elements (411 to 415) are made of the same or different conductive materials.

## Revendications

1. Dispositif de capteur servant à détecter des propriétés de milieux fluides dans un contenant, comprenant
- au moins une plaque de base (42) formée à partir d'un matériau isolant, avec une première surface (42a) exposée aux milieux (3),
- plus de deux éléments de capteur (411 à 415) similaires avec respectivement au moins une première et une deuxième électrode (41a, 41b), qui sont disposées de manière isolée l'une de l'autre sur la première surface (42a) de la plaque de base et qui sont baignées d'un flux des milieux (3),
- un système de pilotage (5) servant à piloter les éléments de capteur, et
- dans lequel les plusieurs éléments de capteur (411 à 415) sont disposés les uns par rapport aux autres dans une position spatiale prédéfinie et de manière répartie sur la première surface (42a) et chaque élément de capteur (411 à 415) peut être piloté par l'intermédiaire de lignes de raccordement (43) individuelles,
**caractérisé en ce que**
- les plusieurs éléments de capteur (411 à 415) sont réalisés de manière à présenter des dimensions géométriques différentes, et dans lequel la taille des éléments de capteur (411 à 415) réalisés différemment est étagée d'une manière prédéfinie, et
- le système de pilotage (5) est réalisé afin de piloter tous les éléments de capteur ou groupes des éléments de capteur afin de détecter les propriétés des milieux (3) fluides.

2. Dispositif de capteur selon la revendication 1, dans lequel les divers éléments de capteur (411 à 415) sont formés à partir d'un matériau identique ou d'un matériau conducteur différent.
